(19) Europäisches Patentamt
European Patent Office
Office européen des brevets

(11) Publication number: **0 009 912**
**B1**

(12) # EUROPEAN PATENT SPECIFICATION

(45) Date of publication of patent specification: **22.06.83**

(51) Int. Cl.$^3$: **C 07 D 311/68,**
A 61 K 31/445

(21) Application number: **79301934.0**

(22) Date of filing: **19.09.79**

(54) **Chromanol derivatives, a process for their preparation and pharmaceutical compositions comprising them.**

(30) Priority: **04.10.78 GB 3930378**
**20.10.78 GB 4130678**
**10.01.79 GB 7900901**

(43) Date of publication of application:
**16.04.80 Bulletin 80/8**

(45) Publication of the grant of the patent:
**22.06.83 Bulletin 83/25**

(84) Designated Contracting States:
**BE CH DE FR GB IT LU NL SE**

(56) References cited:
**GB - A - 1 495 526**
**GB - A - 1 548 221**

(73) Proprietor: **BEECHAM GROUP PLC**
**Beecham House Great West Road**
**Brentford Middlesex (GB)**

(72) Inventor: **Evans, John Morris**
**Cata Old House Lane**
**Roydon Essex (GB)**

(74) Representative: **Stott, Michael John et al,**
**European Patent Attorney Beecham Pharmaceuticals Great Burgh Yew Tree Bottom Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Chromanol derivatives, a process for their preparation, and pharmaceutical compositions comprising them

This invention relates to novel chromanol derivatives having vasodilator activity, to a process for their preparation, and to their use in pharmaceutical compositions for treating hypertension.

GB—A—1 495 526 and GB—A—1 548 221 disclose a large group of derivatives of trans-3-hydroxy-4-aminochroman and state that these compounds may be used in the treatment of hypertension.

It has now been found that 6-cyano-3,4-dihydro-2,2-dimethyl-trans-4-pyrrolidino-2H-benzo[b]pyran-3-ol (and especially the (+) isomer thereof) and certain derivatives thereof may also be used in the treatment of hypertension, and that these compounds have an advantageous therapeutic ratio as judged by the separation of the desired vasodilatory effects from undesired cardiac effects.

It should be noted that GB—A—1 511 187 discloses that 6-cyano-3,4-dihydro-2,2-dimethyl-trans-4-piperidino-2H-benzo[b]pyran-3-ol is a useful chemical intermediate, but in no way suggests any pharmaceutical activity for the compound.

Accordingly, the present invention provides the compounds of the formula (I):

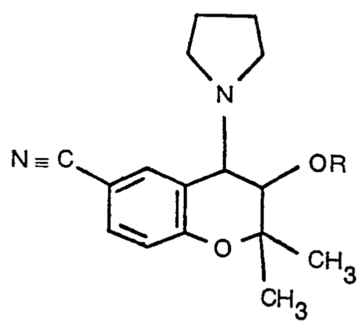

(I)

wherein the pyrrolidino and OR moieties are trans and wherein R is a hydrogen atom, an alkyl group of 1 to 3 carbon atoms or an acyl group of 1 to 8 carbon atoms; or a pharmaceutically acceptable acid addition salt thereof.

Suitable alkyl groups R in relation to formula (I) are the methyl, ethyl and n-propyl groups of which the methyl group is most suitable. Suitable acyl groups R in relation to formula (I) are unsubstituted carboxylic acyl groups as unsubstituted aliphatic acyl or benzoyl.

Preferably R in relation to formula (I) is a hydrogen atom.

The compounds of the formula (I) may be provided in the form of a mixture of stereoisomers or as a single stereoisomer. It is particularly convenient to prepare and use the compounds of the formula (I) as racemic mixtures. Most of the desirable biological activity of the compounds of the formula (I) is found in the (+)-isomer of the compound wherein R is hydrogen and in the derivatives thereof wherein R is alkyl or acyl. Thus in a preferred aspect this invention provides such (+)-isomers (that is when effectively free of the (—)-isomer).

Suitable acid addition salts of the compounds of the formula (I) are those with pharmaceutically acceptable inorganic or organic acids such as hydrochloric, hydrobromic, phosphoric, sulphuric, methanesulphonic, toluenesulphonic, acetic, propionic, succinic, citric, tartaric, mandelic, lactic, gluconic or the like acid.

The present invention also provides a process for the preparation of a compound of the formula (I) or a pharmaceutically acceptable salt thereof which process comprises the reaction of the compound of the formula (II):

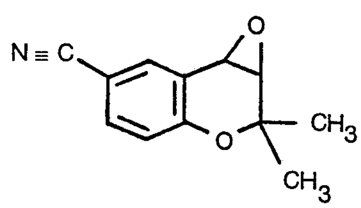

(II)

with pyrrolidine to produce a compound of the formula (I) wherein R is a hydrogen atom, and thereafter optionally alkylating or acylating this compound to give a corresponding compound of the formula (I) wherein R is alkyl or acyl; optionally resolving any compound of the formula (I) so prepared; and/or optionally salifying any compound of the formula (I) so prepared.

The trans-isomer is specifically formed.

The reaction of the epoxide may be carried out at any non-extreme low, medium or high temperature (for example, −10°C to 200°C) but in general ambient or slightly elevated temperatures are most suitable (for example 12° to 100°C). The reaction is normally carried out in a solvent such as

a lower alcohol or lower ketone, for example methanol, ethanol, propanol, acetone or methylethylketone. It has been found that the reaction proceeds smoothly if carried out in refluxing ethanol.

The desired product may be obtained from the reaction mixture by removal of the solvent which is normally accomplished by evaporation under reduced pressure. The initial product may contain some epoxide. This may be separated by dissolving the reaction product in ethyl acetate and extracting into dilute acid. If desired the solvent may be evaporated at this stage but it is usually more convenient to basify, back extract into ethyl acetate and recover by evaporation at reduced pressure. If a salt is desired this product (the free base) may be dissolved in diethyl ether containing a little ethanol and treated with a solution of the acid for example in diethyl ether. The desired salt may then be collected by filtration.

The compound of the formula (II) may be prepared and reacted in situ, for example from a corresponding bromohydrin.

Etherification of the initially produced compound of the formula (I) wherein R is a hydrogen atom may be effected in conventional manner such as reaction with an alkyl iodide in the presence of a base such as potassium tert-butoxide in an inert solvent such as toluene.

Preparation of esters of the compounds of formula (I) wherein R is hydrogen may be by such conventional methods of esterification as reaction with an acylating agent optionally in the presence of an acid acceptor. Suitable acylating agents include acid halides such as a bromide or chloride, an acid in the presence of a condensation promoting agent such as dicyclohexylcarbodiimide or its chemical equivalent, or an acid anhydride. Such reactions are generally carried out in a non-hydroxylic solvent at a non-extreme temperature.

Resolution of a compound of the formula (I) may be effected by forming a salt with an optically active acid such as optically active tartaric acid and fractionally crystallising from a suitable solvent such as ethanol.

In a further aspect the product invention also provides a pharmaceutical composition which comprises a compound of this invention and a pharmaceutically acceptable carrier.

The compositions of this invention are most suitably adapted for oral administration although adaptation for other modes of administration such as by injection for example by intravenous injection for heart failure are also suitable.

In order to obtain consistency of administration it is preferred that the compositions of this invention are in the form of a unit-dose. Suitable unit dose forms include tablets, capsules and powders in sachets or vials. Such unit dose forms aptly contain from 1 to 100 mg of the compound of this invention and more usually from 2 to 50 mg, for example 5 to 25 mg such as 6, 10, 15 or 20 mg. Such compositions may be administered from 1 to 6 times a day, more usually from 2 to 4 times a day, in a manner such that the daily dose is from 5 to 200 mg for a 70 kg human adult and more aptly from 10 to 100 mg.

Shaped oral dosage compositions are favoured composition aspects.

The compositions of this invention may be formulated in conventional manner, for example in a manner similar to that used for known antihypertensive agents such as hydrallazine.

In addition such compositions may contain further active agents such as other anti-hypertensive agents, diuretics and β-blocking agents.

The following Examples illustrate the invention.

Example 1

6-Cyano-3,4-dihydro-2,2-dimethyl-*trans*-4-pyrrolidino-2H-benzo[b]-pyran-3-ol hydrochloride

(a) 4-Cyanophenol (19.60 g), sodium hydroxide (9.90 g), 40% benzyltrimethylammonium hydroxide in methanol (34.50 g) and 3-methyl-3-chlorobutyne (25.50 g) were stirred in water (150 ml) and dichloromethane (150 ml) for 5.5 days at room temperature. After separation of the layers, the aqueous layer was extracted twice with chloroform, and the combined organic phase evaporated leaving a gum which was taken up in ether and washed three times with 10% sodium hydroxide solution and with water before drying over magnesium sulphate. Removal of drying agent and solvent gave a viscous liquid having absorptions in the IR (film) at 2100, 2220, 3290 $cm^{-1}$. This liquid (20.91 g) was heated in o-dichlorobenzene (40 ml) at reflux temperature for 1.5 hours under nitrogen. After distillation of the solvent the fraction boiling at 110—114°/0.02 mmHg (16.57 g) was collected, which on standing formed a low melting solid, having an IR absorption at 2230 $cm^{-1}$. (See M. Harfenist and E. Thom, *J. Org. Chem.*, *37*, 841 (1972) who quote m.p. 36—37°).

Addition to this 6-cyanochromene (16.50 g) dissolved in dimethyl sulphoxide (150 ml) containing water (3.24 ml), of N-bromosuccinimide (31.90 g) with vigorous stirring and cooling, followed by dilution with water and extraction *via* ethyl acetate gave a mixture which was boiled in acetone (300 ml) and water (100 ml) for 5 hours to hydrolyse the small amount of 3,4-dibromide present. Evaporation of solvents gave 6-cyano-*trans*-3-bromo-3,4-dihydro-2,2-dimethyl-2H-benzo[b]pyran-4-ol as white crystals (24.37 g). A small sample had m.p. 128—128.5° from 60—80° petroleum ether, nmr ($CDCl_3$) $\delta$ 1.43 (3H), 1.62 (3H), 3.48 (1H, exchangeable) 4.07 (1H, d, J=9), 4.87 (1H, d, J=9), 6.80 (1H, d, J=8), 7.43 (1H, q, J=8, 2), 7.78 (1H, d, J=2). *Anal.* Calcd. for $C_{12}H_{12}NO_2Br$: C, 51.07; H, 4.26; N, 4.96; Br, 28.37. Found: C, 50.95; H, 4.38; N, 5.03; Br, 28.39%.

(b) The bromohydrin (24.30 g) was stirred with sodium hydroxide pellets (5.00 g) in water (250 ml) and dioxan (200 ml) for 3 hours at room temperature. The solvents were removed by distillation under high vacuum and the residue taken up in ether and washed with water and brine before drying over magnesium sulphate. Removal of drying agent and solvent and gave crude 6-cyano-3,4-dihydro-2,2-dimethyl-3,4-epoxy-2H-benzo[b]pyran (16.02 g) as a gum, having an absorption at 2230 $cm^{-1}$ in the IR and Nmr ($CCl_4$) $\delta$ 1.26 (3H), 1.54 (3H), 3.40 and 3.80 (each 1H, d, J=4), 6.77 (1H, d, J=8), 7.43 (1H, q, J=8, 2), 7.58 (1H, d, J=2).

(c) This epoxide (16.00 g) and pyrrolidine (7.20 ml) were refluxed in ethanol (240 ml) for 3.5 hours. Removal of solvent, addition of ethyl acetate, and washing with water was followed by extraction with 5N hydrochloric acid. The acidic extract was basified with 10N sodium hydroxide solution and extraction via ethyl acetate gave a gum which was crude 6-cyano-3,4-dihydro-2,2-dimethyl-*trans*-4-pyrrolidino-2H-benzo[b]pyran-3-ol. This compound was then taken up in diethyl ether containing a little ethanol and treated with ethereal hydrogen chloride. The precipitate was collected and washed with diethyl ether leaving 6-cyano-3,4-dihydro-2,2-dimethyl-*trans*-4-pyrrolidino-2H-benzo[b]pyran-3-ol hydrochloride as a white solid (11.02 g), m.p. 202—204°; IR absorption at 2220 $cm^{-1}$; nmr ($CDCl_3$) $\delta$ 1.19 (3H), 1.73 (3H), 2.22 (4H, broad m), 3.13 (2H, broad m), 3.97 (2H, broad m), 4.20 (1H, d, J=8), 4.86 (1H, d, J=8), 5.58 (s, 1 exchangeable H, broad), 6.87 (1H, d, J=8), 7.47 (1H, q, J=8, 2), 8.72 (1H, d, J=2). *Anal.* Calcd. for $C_{16}H_{21}N_2O_2Cl$: C, 62,23; H, 6.86; N, 9.07; Cl, 11.48. Found: C, 62,34; H, 6.73; N, 8.82; Cl, 11.40%.

Example 2

6-Cyano-3,4-dihydro-2,2-dimethyl-*trans*-4-pyrrolidino-2H-benzo[b]pyran-3-yl 2,2-dimethylpropanoate

To a solution of 4-dimethylaminopyridine (0.98 g) in dichloromethane (50 ml) was added 2,2-dimethylpropanoyl chloride (0.69 ml) dropwise and with gentle stirring, followed by crude 6-cyano-3,4-dihydro-2,2-dimethyl-*trans*-4-pyrrolidino-2H-benzo[b]pyran-3-ol (2.50 g) in dichloromethane (50 ml) during 4 minutes. The resulting red solution was heated under reflux for 40 hours before cooling and evaporation of solvent. The orange residue was taken up in ethyl acetate and washed with water, dried and evaporated leaving a mustard coloured solid (2.97 g) which was separated by chromatography on silica gel (110 g) with mixtures of ethyl acetate and 60—80° petroleum ether using a gradient elution technique, into crude ester (1.12 g) and starting material (1.13 g). Recrystallisation of the crude material from 60—80° petroleum ether gave 6-cyano-3,4-dihydro-2,2-dimethyl-*trans*-4-pyrrolidino-2H-benzo[b]pyran-3-yl 2,2-dimethylpropanoate (0.73 g) as white crystals of m.p. 109—110°C; IR absorptions at 1730, 2220 $cm^{-1}$; nmr ($CDCl_3$) $\delta$ 1.24 (9H), 1.29 (3H), 1.41 (3H), 1.75 (4H, broad m), 2.73 (4H, broad m), 4.09 (1H, d, J=9), 5.33 (1H, d, J=9), 6.86 (1H, d, J=8), 7.43 (1H, q, J=8, 2), 7.74 (1H, d, J=2), *Anal.* Calcd. for $C_{21}H_{28}N_2O_3$: C, 70.76; H, 7.92; N, 7.86. Found: C, 70.74; H, 8.03; N, 7.76%.

Example 3

6-Cyano-3,4-dihydro-2,2-dimethyl-*trans*-3-methoxy-4-pyrrolidino-2H-benzo[b]pyran

To potassium t-butoxide (1.03 g) in dry toluene (40 ml) was added dropwise with stirring 6-cyano-3,4-dihydro-2,2-dimethyl-*trans*-4-pyrrolidino-2H-benzo[b]pyran-3-ol (2.50 g) in toluene (100 ml) under nitrogen. After 10 minutes, methyl iodide (0.62 ml) in toluene (20 ml) was added dropwise, and the resulting yellow reaction mixture was stirred at 75—80°C for 16 hours. Cooling, and cautious addition of water, separation of the organic layer, washing with water, drying and evaporation gave a red gum (2.66 g) which was separated, by column chromatography on silica gel (110 g) with mixtures of ethyl acetate and 60—80° petroleum ether using a gradient elution technique, into crude ether (1.34 g) and starting material (0.91 g). One crystallisation from 60—80° petroleum ether gave 6-cyano-3,4-dihydro-2,2-dimethyl-*trans*-3-methoxy-4-pyrrolidino-2H-benzo[b]pyran (1.06 g) as off-white crystals m.p. 108—109°C; nmr ($CDCl_3$) $\delta$ 1.22 (3H), 1.50 (3H), 1.80 (4H, broad m), 2.76 (4H, broad m), 3.39 (1H, d, J=9), 3.51 (3H), 3.98 (1H, d, J=9), 6.76 (1H, d, J=8), 7.34 (1H, q, J=8, 2), 7.72 (1H, d, J=2). *Anal.* Calcd. for $C_{17}H_{22}N_2O_2$: C, 71.30; H, 7.74; N, 9.78. Found: C, 71.12; H, 7.96; N, 9.72%.

Example 4

Resolution of 6-cyano-3,4-dihydro-2,2-dimethyl-*trans*-4-pyrrolidino-2H-benzo-[b]-pyran-3-ol

Racemic 6-cyano-3,4-dihydro-2,2-dimethyl-*trans*-4-pyrrolidino-2H-benzo[b]-pyran-3-ol (4.12 g) and (+)-Tartaric acid (2.55 g) both dissolved in ethanol, were combined and the resulting solution evaporated leaving a cream foam. Three recrystallisations from ethanol gave a tartrate (1.01 g) of mp 173—173.5° and an $[\alpha]$=—64°. Basification with $NaHCO_3$ and extraction with diethyl ether gave the minus isomer of the free base (0.64 g) of $[\alpha]_{EtOH}$=—101°. Treatment of an ethereal solution of this free base with ethereal-anhydrous HCl and one recrystallisation from ethanol-diethyl ether gave (—)-6-cyano-3,4-dihydro-2,2-dimethyl-*trans*-4-pyrrolidino-2H-benzo-[b]-pyran-3-ol hydrochloride (0.49 g) of mp 184—185°C, $[\alpha]$=—98°C Anal. Calcd. for $C_{16}H_{21}N_2O_2Cl$: C, 62.23; H, 6.85; H, 9.07; Cl, 11.48. Found: C, 62.29; H. 7.11; N, 9.17; Cl, 11.39%.

The mother liquors remaining after the three recrystallisations of the tartrate prepared from the

racemic free base and (+)-tartaric acid were evaporated to dryness, dissolved in water, and basified with $NaHCO_3$. Extraction via diethyl ether gave crude free base (3.10 g) which was treated with (—)-tartaric acid (1.67 g) in ethanol. Evaporation gave a cream foam (4.78 g).

Four recrystallisations from ethanol gave a tartrate (1.42 g) of mp 172.5—173.5° and $[\alpha]+58°$. Basification with $NaHCO_3$ and extraction with diethyl ether gave the plus isomer of the free base (0.88 g) of $[\alpha]_{EtOH}=+115°$. Treatment of an ethereal solution of this free base with ethereal-anhydrous HCl and one recrystallisation from ethanol-diethyl ether gave (+)-6-cyano-3,4-dihydro-2,2-dimethyl-*trans*-4-pyrrolidino-2H-benzo-[b]-pyran-3-ol hydrochloride (0.87 g) of mp 175—177°C, $[\alpha]=+100°$. Anal. Calcd. for $C_{16}H_{21}N_2O_2Cl$: C, 62.23; H, 6.85; N, 9.07; Cl, 11.48. Found: C, 61.94; H, 7.06; N, 9.28; Cl 11.63%.

(Optical rotations were determined in water unless otherwise stated and measured at the sodium-D line).

Example 5

6-Cyano-3,4-dihydro-2,2-dimethyl-*trans*-4-pyrrolidino-2H-benzo-[b]-pyran-3-ol hydrochloride

6-Cyano-*trans*-3-bromo-3,4-dihydro-2,2-dimethyl-2H-benzo[b]pyran-4-ol (1.57 g) was dissolved in pyrrolidine (2.0 ml) and the solution heated under reflux for 25 min. After cooling, the solution was subjected to reduced pressure to remove traces of pyrrolidine. The residual gum was dissolved in ethyl acetate and washed with aqueous sodium carbonate solution, and water, before extraction with 1N hydrochloric acid. The organic layer was discarded, and the aqueous layer was basified with 2.5 N sodium hydroxide and extracted with ethyl acetate. Water washing, drying, and evaporation of the organic layer gave the crude 6-cyano-3,4-dihydro-2,2-dimethyl-*trans*-4-pyrrolidino-2H-benzo[b]pyran-3-ol (0.85 g) having identical chromatographic characteristics to that described in Example 1. The hydrochloride salt was then prepared in the same manner as described in Example 1.

Demonstration 1—Effectiveness of the compound of Example 1

Systolic blood pressures were recorded by a modification of the tail cuff method described by I. M. Claxton, M. G. Palfreyman, R. H. Poyser and R. L. Whiting, *European Journal of Pharmacology*, *37*, 179 (1976). An oscilloscope or W+W BP recorder, model 8002, was used to displace pulses. Prior to all measurements rats were placed in a heated environment (33.5±0.5°C) before transfer to a restraining cage. Each determination of blood pressure was the mean of at least 6 readings. Spontaneously hypertensive rats (ages 12—18 weeks) with systolic blood pressures>170 mmHg were considered hypertensive.

| Compound of example 1 | Time post dose hours | % change in systolic blood pressure | % change in heart rate |
|---|---|---|---|
| 5 Rats<br>Dose 1 mg/kg p.o. | 1 | −22±2 | +10±3 |
| Initial blood pressure<br>208±3 | 2 | −21±2 | +8±2 |
| Initial heart rate | 4 | −29±2 | +7±2 |
| 471±11 | 6 | −21±2 | +7±2 |
| | 24 | −18±2 | −4±4 |
| 6 Rats<br>Dose 0.3 mg/kg p.o. | 1 | −21±2 | +2±1 |
| Initial blood pressure<br>197±2 | 2 | −12±2 | −8±2 |
| Initial heart rate | 4 | −8±2 | −6±1 |
| 477±5 | 6 | −2±2 | −4±1 |
| | 24 | +6±4 | −4±4 |

Demonstration 2—Effectiveness of the compound of Example 4

Systolic blood pressures were recorded by a modification of the tail cuff method described by I. M. Claxton, M. G. Palfreyman, R. H. Poyser and R. L. Whiting, European Journal of Pharmacology, *37*, 179 (1976). A W and W B.P. recorder, model 8002, was used to display pulses. Prior to all measurements, DOCA-Salt treated hypertensive rats were placed in a heated environment (33.5±0.5°C) before transfer to a restraining cage. Each determination of b.p. was the mean of at least 6 readings.

| (−)-Isomer | Time post dose hours | % change in systolic B.P. | % change in heart rate |
|---|---|---|---|
| 5 rats | Initial values | 185±88 mmHg | 344±17 beats/min. |
| 1 mg/kg p.o. | 1 | −3±3 | 16±7 |
| | 2 | −4±3 | 10±7 |
| | 4 | −5±3 | 2±6 |
| | 6 | −1±2 | 2±2 |
| | 24 | 9±1 | 17±6 |

| (+)-Isomer | Time post dose hours | % change in systolic B.P. | % change in heart rate |
|---|---|---|---|
| 6 rats | Initial values | 191±4 mmHg | 331±13 beats/min. |
| 1 mg/kg p.o. | 1 | −37±2 | 22±3 |
| | 2 | −27±3 | 17±5 |
| | 4 | −23±4 | 7±6 |
| | 6 | −14±3 | 13±4 |
| | 24 | 4±3 | 9±3 |

**Claims**

1. A compound of the formula (I):

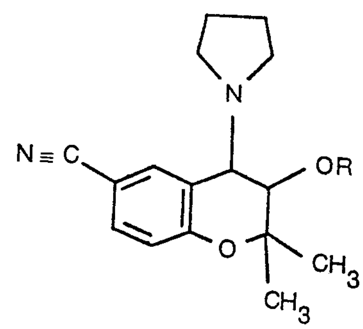

(I)

wherein the pyrrolidino and OR moieties are trans and wherein R is a hydrogen atom, an alkyl group of 1 to 3 carbon atoms or an acyl group of 1 to 8 carbon atoms; or a pharmaceutically acceptable acid addition salt thereof.

2. 6-Cyano-3,4-dihydro-2,2-dimethyl-*trans*-4-pyrrolidino-2H-benzo[b]pyran-3-ol or a pharmaceutically acceptable acid addition salt thereof.

3. 6-Cyano-3,4-dihydro-2,2-dimethyl-*trans*-4-pyrrolidino-2H-benzo[b]pyran-3-ol hydrochloride.

4. 6-Cyano-3,4-dihydro-2,2-dimethyl-*trans*-4-pyrrolidino-2H-benzo[b]pyran-3-yl 2,2-dimethylpropanoate or a pharmaceutically acceptable acid addition salt thereof.

5. 6-Cyano-3,4-dihydro-2,2-dimethyl-*trans*-3-methoxy-4-pyrrolidino-2H-benzo[b]pyran or a pharmaceutically acceptable acid addition salt thereof.

6. A compound as claimed in claim 1 having the stereochemistry of (+)-6-cyano-3,4-dihydro-2,2-dimethyl-*trans*-4-pyrrolidino-2H-benzopyran-3-ol.

7. (+)-6-Cyano-3,4-dihydro-2,2-dimethyl-*trans*-4-pyrrolidino-2H-benzo[b]pyran-3-ol or a pharmaceutically acceptable salt thereof.

8. (+)-6-Cyano-3,4-dihydro-2,2-dimethyl-*trans*-4-pyrrolidino-2H-benzo[b]pyran-3-ol hydrochloride.

9. A pharmaceutical composition comprising a compound as claimed in any of claims 1 to 8 and a pharmaceutically acceptable carrier.

10. A composition as claimed in claim 9 in the form of an orally administrable shaped unit dose form containing from 2 to 50 mg of said compound as claimed in any of claims 1 to 8.

11. A compound as claimed in any of claims 1 to 9, for use as a vasodilatory anti-hypertensive agent.

12. A process for the preparation of a compound as claimed in Claim 1, which process comprises reacting the compound of the formula (II):

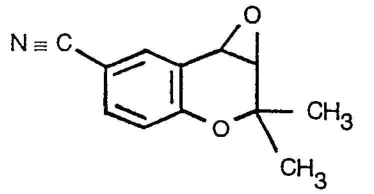

(II)

with pyrrolidine to produce a compound of the formula (I) wherein R is a hydrogen atom; and thereafter optionally alkylating or acylating this compound to give a corresponding compound of the formula (I) wherein R is alkyl or acyl; optionally resolving any compound of the formula (I) so prepared; and/or optionally satisfying any compound of the formula (I) so prepared.

**Patentansprüche**

1. Verbindung der Formel (I)

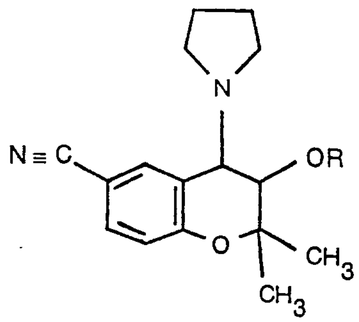

(I)

in der der Pyrrolidinorest und der Rest -OR in trans-Stellung stehen und R ein Wasserstoffatom, ein Alkylrest mit 1 bis 3 Kohlenstoffatomen oder ein Acylrest mit 1 bis 8 Kohlenstoffatomen ist, oder deren pharmakologisch verträgliches Säureadditionssalz.

2. 6-Cyano-3,4-dihydro-2,2-dimethyl-trans-4-pyrrolidino-2H-benzo[b]pyran-3-ol oder dessen pharmakologisch verträgliches Säureadditionssalz.

3. 6-Cyano-3,4-dihydro-2,2-dimethyl-*trans*-4-pyrrolidino-2H-benzo[b]pyran-3-ol-hydrochlorid.

4. 2,2-Dimethylpropionsäureester des 6-Cyano-3,4-dihydro-2,2-dimethyl-*trans*-4-pyrrolidino-2H-benzo[b]pyran-3-ols oder dessen pharmakologisch verträgliches Säureadditionssalz.

5. 6-Cyano-3,4-dihydro-2,2-dimethyl-trans-3-methoxy-4-pyrrolidino-2H-benzo[b]pyran oder dessen pharmakologisch verträgliches Säureadditionssalz.

6. Verbindung nach Anspruch 1 mit der Stereochemie des (+)-6-Cyano-3,4-dihydro-2,2-dimethyl-*trans*-4-pyrrolidino-2H-benzopyran-3-ols.

7. (+)-6-Cyano-3,4-dihydro-2,2-dimethyl-*trans*-4-pyrrolidino-2H-benzo[b]pyran-3-ol oder dessen pharmakologisch verträgliches Salz.

8. (+)-6-Cyano-3,4-dihydro-2,2-dimethyl-*trans*-4-pyrrolidino-2H-benzo[b]pyran-3-ol-hydrochlorid.

9. Pharmazeutische Zusammensetzung, enthaltend eine Verbindung nach irgendeinem der Ansprüche 1 bis 8 und ein pharmakologisch verträgliches Trägermaterial.

10. Zusammensetzung nach Anspruch 9 in Form einer geformten, oral verabreichbaren Einzeldosierungsform, die 2 bis 50 mg einer Verbindung nach irgendeinem der Ansprüche 1 bis 8 enthält.

11. Verbindung nach irgendeinem der Ansprüche 1 bis 9 zum Gebrauch als gefäßerweiterndes blutdrucksenkendes Mittel.

12. Verfahren zur Herstellung einer Verbindung nach Anspruch 1, das die Maßnahme einer Umsetzung einer Verbindung der Formel (II)

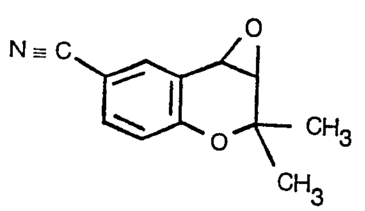

(II)

mit Pyrrolidin zur Herstellung einer Verbindung der Formel (I), in der R ein Wasserstoffatom ist, gegebenenfalls anschließend die Maßnahme einer Alkylierung oder Acylierung dieser Verbindung zur Herstellung einer entsprechenden Verbindung der Formel (I), in der R ein Alkyl- oder Acylrest ist, gegebenenfalls die Maßnahme eines Auflösens irgendeiner der derart hergestellten Verbindung der Formel (I) und/oder gegebenenfalls die Maßnahme einer Umwandlung irgendeiner der derart hergestellten Verbindungen der Formel (I) in ein Salz umfaßt.

**Revendications**

1. Composé de formule (I):

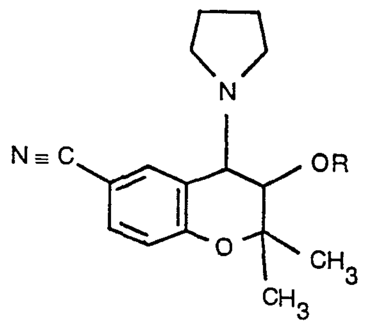

(I)

dans laquelle les fractions molaires pyrrolidino et OR sont en position trans et dans laquelle R est un atome d'hydrogène, un groupe alcoyle ayant de 1 à 3 atomes de carbone ou un groupe acyle ayant de 1 à 8 atomes de carbone, ou sel d'addition avec un acide pharmaceutiquement acceptable de ce composé.

2. Cyano-3,4-dihydro-2,2-diméthyl-trans-4-pyrrolidino-2H-benzo-[b]pyranne-3-ol ou sel d'addition avec un acide pharmaceutiquement acceptable de ce composé.

3. Chlorhydrate de 6-cyano-3,4-dihydro-2,2-di-méthyl-trans-4-pyrrolidino-2H-benzo-[b]pyranne-3-ol.

4. 2,2-Diméthyl-propanoate de 6-cyano-3,4-dihydro-2,2-diméthyl-trans-4-pyrrolidino-2H-benzo-[b]pyranne-3-yle ou sel d'addition avec un acide pharmaceutiquement acceptable de ce composé.

5. 6-Cyano-3,4-dihydro-2,2-diméthyl-trans-3-méthoxy-4-pyrrolidino-2H-benzo-[b]pyranne ou sel d'addition avec un acide pharmaceutiquement acceptable de ce composé.

6. Composé suivant la revendication 1, ayant la stéréochimie du (+)-6-cyano-3,4-dihydro-2,2-diméthyl-trans-4-pyrrolidino-2H-benzopyranne-3-ol.

7. (+)-6-Cyano-3,4-dihydro-2,2-diméthyl-trans-4-pyrrolidino-2H-benzo-[b]pyranne-3-ol ou sel pharmaceutiquement acceptable de celui-ci.

8. Chlorhydrate de (+)-6-cyano-3,4-dihydro-2,2-diméthyl-trans-4-pyrrolidino-2H-benzo-[b]pyranne-3-ol.

9. Composition pharmaceutique renfermant un composé suivant l'une quelconque des revendications 1 à 8 et un véhicule ou excipient pharmaceutiquement acceptable.

10. Composition suivant la revendication 9, se présentant sous la forme d'une dose unitaire pouvant être administrée oralement, contenant de 2 à 50 mg de ce composé suivant l'une quelconque des revendications 1 à 8.

11. Composé suivant l'une quelconque des revendications 1 à 9, destiné à être utilisé comme agent anti-hypertenseur vasodilatateur.

12. Procédé pour la préparation d'un composé suivant la revendication 1, ce procédé consistant à faire réagir le composé de formule (II):

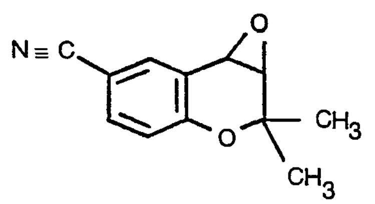

(II)

avec de la pyrrolidine, pour produire un composé de formule (I) dans laquelle R est un atome d'hydrogène, puis si désiré à effectuer l'alcoylation ou l'acylation de ce composé pour donner un composé correspondant de formule (I) dans laquelle R est un groupe alcoyle ou acyle, si désiré à dédoubler un composé quelconque de formule (I) ainsi préparé et/ou si désiré à salifier un composé quelconque de formule (I) ainsi préparé.